# EUROPEAN PATENT APPLICATION

(11) **EP 4 141 115 A1**
(43) Date of publication of application: **01.03.2023**
(21) Application number: 21793509.7
(22) Date of filing: 20.04.2021
(51) Int. Cl.: C12N 15/09, C12Q 1/6883

(54) **NUCLEIC ACID DETECTION METHOD AND OLIGONUCLEOTIDE PROBE**

(30) Priority: 22.04.2020 JP 2020076369
(71) Applicant: Nicca Chemical Co., Ltd., Fukui-shi, Fukui 910-8670 (JP)
(72) Inventor: OKABE, Ayako, Fukui-shi, Fukui 910-8670 (JP); NAKASHA, Ayaka, Fukui-shi, Fukui 910-8670 (JP); NORIKI, Sakon, Fukui-shi, Fukui 910-8507 (JP)
(74) Representative: J A Kemp LLP
(86) International application number: PCT/JP2021/015991
(87) International publication number: WO 2021/215428

(57) **Abstract**

A method of detecting a target sequence is provided, the method comprising: hybridizing by bringing an oligonucleotide probe into contact with a nucleic acid present in a test sample, wherein the oligonucleotide probe has a hybridization sequence with respect to a target sequence of the nucleic acid and has at least one covalent bonding group that is crosslinkable with a target base in the target sequence by light irradiation when being specifically hybridized with the target sequence in the hybridization sequence, thereby forming an authentic hybridization product; irradiating light to the test sample after the hybridizing, wherein the covalent bonding group in the authentic hybridization product and the target sequence are crosslinked, thereby forming the authentic hybridization product having a crosslinked structure; and denaturating the test sample after the irradiating by applying a denaturating condition in which the authentic crosslinked hybridization product is able to be maintained and a non-specific hybridization product is able to be dissociated and separating the oligonucleotide probe derived from the non-specific hybridization product.

## Description

### Technical Field

### Cross Reference to Related Applications

This application is related to Japanese Patent Application No. 2020-76369 filed on April 22, 2020 and claims priority to the Japanese application entire contents of which are incorporated by reference herein. The present teachings relate to a nucleic acid detection method and oligonucleotide probe.

### Background Art

Oligonucleotide probes have become useful diagnostic tools for diseases in which gene mutations or amplifications are observed. For example, hybridization with DNA, mRNA or the like extracted from specimens such as cells or tissues is useful for diagnosis of infectious diseases, tumors, and the like, which can be diagnosed by detecting such DNA (Patent Literature 1).

Hybridization, although being a useful method, still has various problems. For example, if hybridization is performed at a low temperature in order to obtain a sufficiently high hybridization efficiency, the specificity of hybridization decreases. On the other hand, if hybridization is performed at a higher temperature, the specificity is improved but the sensitivity may decrease. As one solution, currently. a probe with a high Tm has been designed in order to achieve securing of both of hybridization efficiency and hybridization specificity. In addition, one method for improving specificity is to perform sufficient washing under conditions of high stringency after hybridization.

### Summary

However, elongation of the probe length in order to raise the Tm temperature causes a problem of lowering the specificity to a target sequence. In addition, even if washing under high stringency conditions is used. non-specific hybridization products remain or specific hybridization products are washed away, which may result in a significant reduction in the accuracy of disease examination. On the other hand. it is not easy to set stringency conditions under which only specific hybridization products remain. In addition, peptide nucleic acids (PNA) have been developed to raise the Tm temperature, but even if peptide nucleic acids are used, washing under high stringency conditions is difficult.

From the above, it has been difficult to improve the examination accuracy and secure the reproducibility by combining low stringency during hybridization and high stringency during washing by raising the Tm temperature of the probe.

In an examination by hybridization, for example, in situ hybridization, there is a need to establish a nucleic acid detection method in which an examination having excellent accuracy and reproducibility is realized without depending largely on conventional stringency adjustment.

The present specification provides a nucleic acid detection method that implements diagnosis with high accuracy and reproducibility. In addition, there is provided an oligonucleotide probe used in such a detection method.

The inventors have found that the above problem can be addressed when a photocrosslinkable functional group is provided in an oligonucleotide probe, a hybridization product crosslinked by photocrosslinking after a hybridization step is acquired, and denaturation conditions for the hybridization product are then applied to a test sample. Based on such findings, the present specification provides the following aspects.
[1] A nucleic acid detection method, comprising:
   hybridizing by bringing an oligonucleotide probe into contact with a nucleic acid present in a test sample, wherein the oligonucleotide probe has a hybridization sequence with respect to a target sequence of the nucleic acid and has at least one covalent bonding group that is crosslinkable with a target base in the target sequence by light irradiation when being specifically hybridized with the target sequence in the hybridization sequence, thereby forming an authentic hybridization product;
   irradiating light to the test sample after the hybridizing, wherein the covalent bonding group in the authentic hybridization product and the target sequence are crosslinked, thereby forming the authentic hybridization product having a crosslinked structure; and
   denaturating the test sample after the irradiating by applying a denaturating condition in which the authentic crosslinked hybridization product is able to be maintained and a non-specific hybridization product is able to be dissociated and separating the oligonucleotide probe derived from the non-specific hybridization product.
[2] The method according to [1].
   wherein the denaturating comprises further promotion of dissociation of hybridization of the authentic hybridization product and separation of the oligonucleotide probe, derived from the authentic hybridization product, and the authentic crosslinked hybridization product.
[3] The method according to [1] or [2],
   wherein the hybridizing comprises performing in situ hybridization.
[4] The method according to [3],
   wherein the hybridizing comprises supplying the oligonucleotide probe into tissue sections collected from a cultured cell, a cancer patient or the like.
[5] The method according to any one of [1] to [4],
   wherein the denaturating comprises denaturation of the test sample with an aqueous solution mixture containing formamide at a concentration exceeding 60 volume% after the hybridizing.
[6] The method according to any one of [1] to [5],
   wherein the denaturating comprises denaturation using a liquid at room temperature or higher.
[7] The method according to any one of [1] to [6].
   wherein the denaturating comprisese denaturation with a denaturation solution containing a denaturant other than formamide.
[8] The method according to [7].
   wherein the denaturant other than formamide is tetramethylammonium chloride.
[9] The method according to any one of [1] to [8].
   wherein the irradiating comprises irradiation of the light having a maximum wavelength of 340 to 380 nm.
[10] The method according to any one of [1] to [9],
   wherein the hybridization sequence has 5 bases or more and 200 bases or less.
[11] The method according to any one of [1] to [10],
   wherein the target sequence comprises an RNA sequence in human HER2 protein mRNA.
[12] The method according to any one of [1] to [10],
   wherein the target sequence comprises an RNA sequence in Satb2 protein mRNA.
[13] The method according to any one of [1] to [10],
   wherein the target sequence comprises an RNA sequence in EBER small RNA.
[14] The method according to any one of [1] to [10].
   wherein the target sequence comprises an RNA sequence in 28S rRNA.
[15] The method according to any one of [1] to [14],
   wherein the denaturating comprises denaturation of the test sample with an aqueous solution mixture containing 80 volume% of formamide after the hybridizing.
[16] An oligonucleotide probe for detecting nucleic acid, comprising
   a hybridization sequence that is able to hybridize with a target sequence of the nucleic acid, and at least one covalent bonding group that is crosslinkable with a target base in the target sequence by light irradiation when being specifically hybridized with the target sequence in the hybridization sequence,
   wherein the hybridization sequence and at least one covalent bonding group are configured such that an authentic cross-linked hybridization product in which the hybridization sequence and the target sequence are hybridized and crosslinked by the light irradiation is maintained but a non-specific hybridization product hybridization product is able to be selectively removed.
[17] A labeled oligonucleotide detection kit including the oligonucleotide probe according to [16] and a denaturant having an effect equal to or greater than that of an aqueous mixed solution mixture containing formamide at a concentration exceeding 60 volume%.

### Brief Description of Drawings

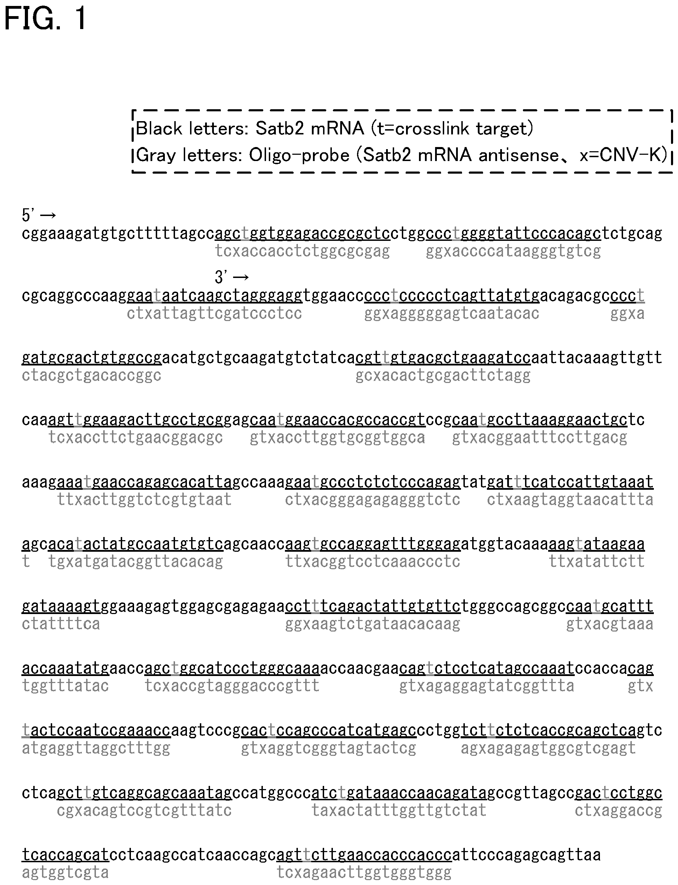

FIG. 1 shows a design of probes for Satb2 protein mRNA;

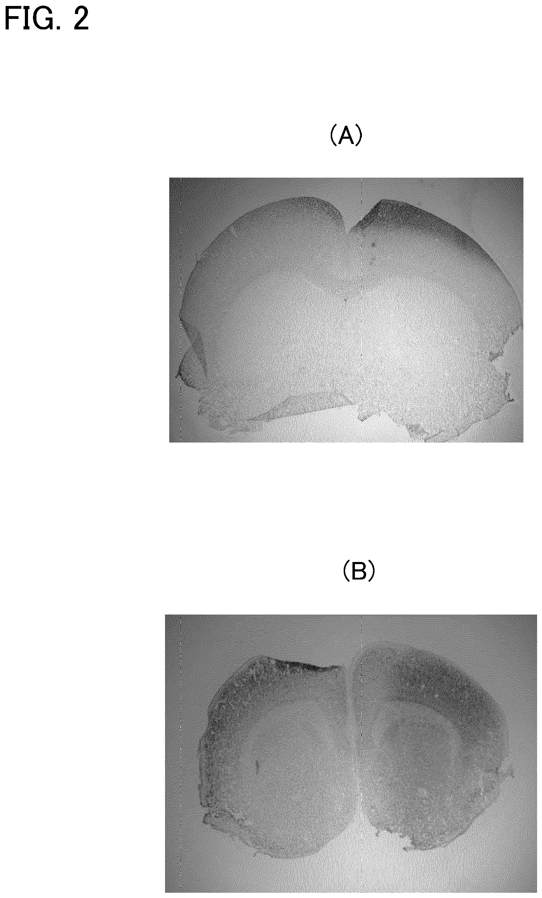

FIG. 2 shows Satb2 protein mRNA CISH in cerebrum sections collected from mice, FIG. 2(A) shows hybridization involving covalent bonds with probes having covalent bonding groups (after denaturation/washing with 80 volume% formamide aqueous solution), and FIG. 2(B) shows hybridizations involving covalent bonds with probes having covalent bonding groups (after denaturation/washing with 80 volume% formamide TBS solution);

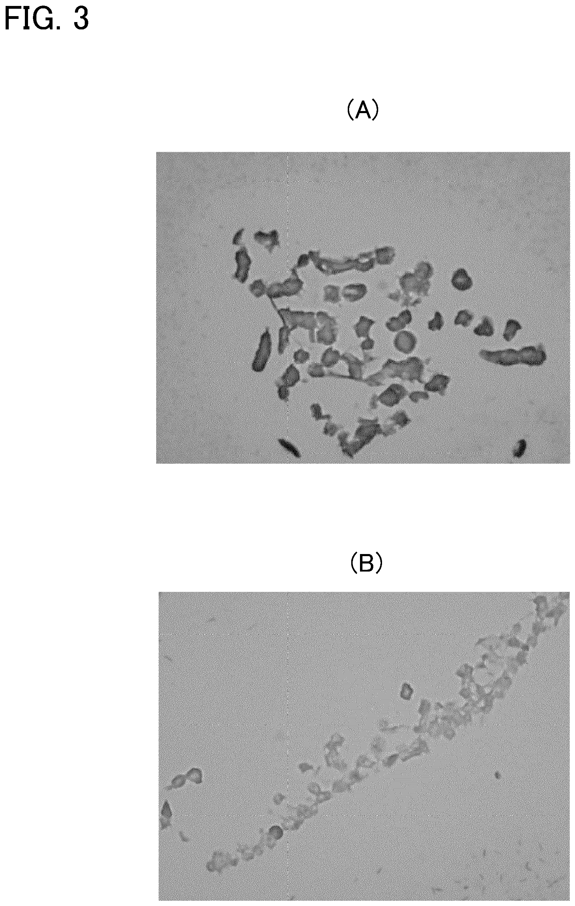

FIG. 3 shows hybridization with HER2 protein mRNA CISH, FIG. 3(A) shows hybridizations involving covalent bonds with probes having covalent bonding groups in HER2 positive cells (after denaturation/washing with 80 volume% formamide aqueous solution), and FIG. 3(B) shows hybridizations involving covalent bonds with probes having covalent bonding groups in HER2 negative cells (after denaturation/washing with 80 volume% formamide aqueous solution);

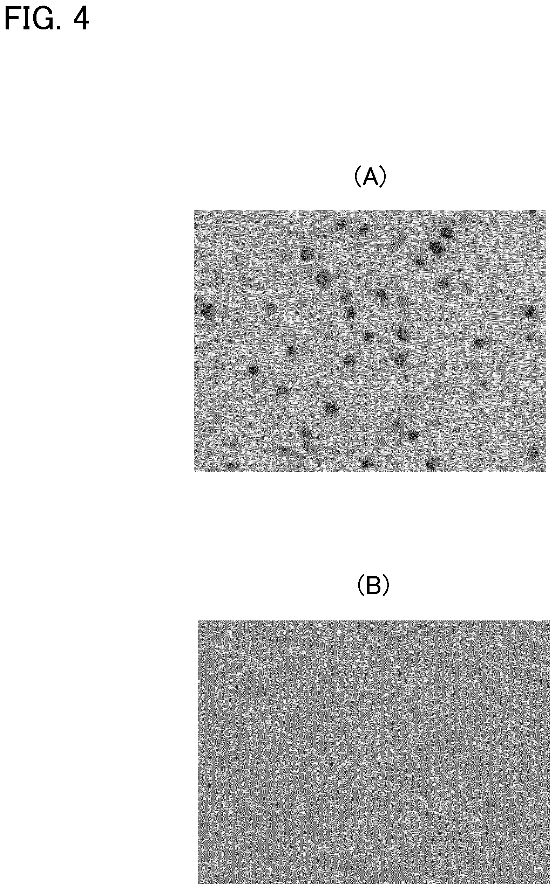

FIG. 4 shows hybridization with EB virus-produced small RNA (EBER) CISH, FIG. 4(A) shows hybridization involving covalent bonds with probes having covalent bonding groups (after denaturation/washing with 80 volume% 2×SSC solution at 55°C), and FIG. 4(B) shows hybridizations involving covalent bonds with probes having no covalent bonding groups (after denaturation/washing with 80 volume% 2×SSC solution at 55°C): and

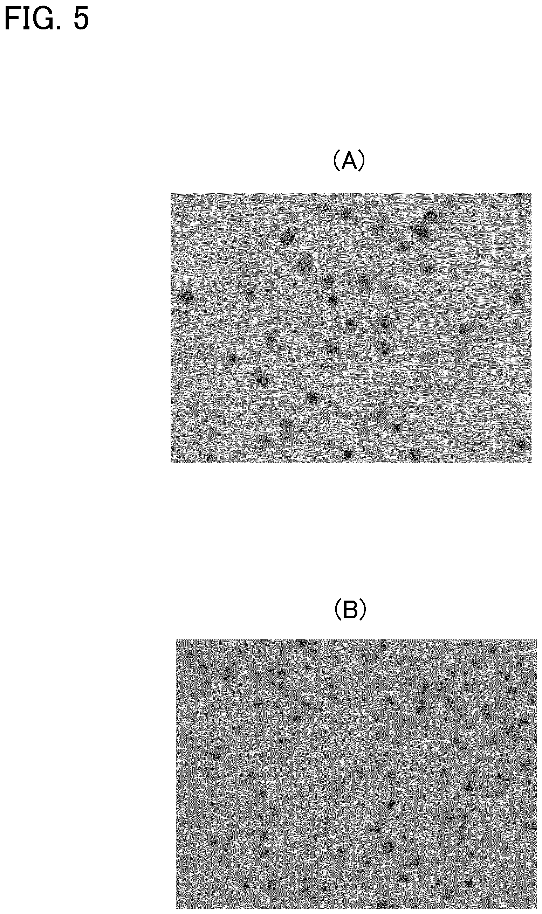

FIG. 5 shows hybridizations with EB virus-produced small RNA (EBER) CISH. FIG. 5(A) shows room temperature hybridization involving covalent bonds with probes having covalent bonding groups (after denaturation/washing with 50 volume% formamide 2×SSC (room temperature)), and FIG. 5(B) shows hybridization at 55°C with commercially available PNA probes (stringent buffer, after denaturation/washing at 55°C).

### Description of Embodiments

The present disclosure relates to a nucleic acid detection method and an oligonucleotide probe. According to the nucleic acid detection method disclosed in this specification (hereinafter simply referred to as this detection method), an authentic crosslinked hybridization products is irreversibly linked to a target sequence with high specificity. Therefore, under denaturation conditions in which an authentic crosslinked hybridization product can be maintained and a non-specific hybridization product can be dissociated, the authentic crosslinked hybridization product can be easily separated from the non-specific hybridization product and detected. That is, according to the denaturation conditions, it is possible to detect a nucleic acid having a target sequence with excellent accuracy and reproducibility without significantly depending on stringency strength. As a result, true positive can be detected with high sensitivity, and true negative can be detected with high specificity.

In the following, representative, non-limitative, specific embodiments of the present disclosure are described in detail with reference to the attached drawings as needed. The detailed description has been provided for the purpose of merely teaching the details required to carry out preferable embodiments of the present invention to a person skilled in the art, and has not been intended to limit the scope of the present disclosure. The additional characteristics and inventions to be disclosed below can be used independently of or in combination with other characteristics and inventions so as to provide a nucleic acid detection method and oligonucleotide probe.

The combinations of characteristics and steps to be disclosed in the following detailed description are not essential for carrying out the present disclosure in its broadest reasonable construction but are given for the purpose of merely describing, in particular, the representative, specific examples of the present disclosure. Besides, the various characteristics of the representative, specific examples described above and below and the various characteristics of the matters described in the independent claims and the dependent claims are not necessarily required to be combined in the same way or in the same order as in the specific examples when providing additional and useful embodiments of the present disclosure.

All the characteristics described in the present specification and/or in the claims are disclosed separately and independently of each other for the same purpose as the purpose of the disclosure at the time of filing of the application and for the purpose of restricting the claimed subject matter, independent of the composition of the characteristics described in the embodiments and/or in the claims. In addition, description regarding any numerical range, any group. and any collection are given so as to disclose every possible intermediate value or intermediate entity for the same purpose as the purpose of the disclosure at the time of filing of the application and for the purpose of restricting the claimed subject matter.

Hereinafter, the nucleic acid detection method, oligonucleotide probe and the like disclosed in this specification will be described in detail.

Here, in this specification, test samples are not particularly limited, and may be any sample for which detection of a target nucleic acid is required. Therefore, the test samples may be any sample that may contain nucleic acids to be detected, and include samples collected from nature or living organisms without change, and also include samples from which DNA or RNA in samples has been extracted from such samples, and samples in which the content of DNA or RNA in such samples has been increased by a known nucleic acid amplification method or the like or reverse transcription has been performed. Examples of test samples include various biological fluid samples such as blood and urine artificially collected or naturally separated from animals such as humans and livestock, cell samples and tissue samples, and also include so-called medical and sanitary specimen samples such as processed samples on which nucleic acid extraction/amplification/reverse transcription and the like have been performed on the above samples. In addition, the test samples include various parts or forms of biological samples or processed samples artificially collected or naturally separated from plants or other organisms. In addition, the test sample includes a sample that is for non-medical purposes and which may contain nucleic acids.

In addition, in this specification, nucleic acids include natural nucleic acids such as DNA and RNA stored in living organisms as well as modified DNA and RNA including modifications with respect to these components as long as they are crosslinkable with the oligonucleotide probe disclosed in this specification. In addition, DNA is not particularly limited as long as it is naturally-derived DNA that living organisms can store or DNA artificially introduced from the outside, and includes DNA on vectors such as genomes and plasmids. In addition, RNA is not particularly limited as long as it is naturally-derived RNA that living organisms can store or RNA introduced from the outside, and includes mRNA, tRNA, rRNA, ncRNA such as siRNA and miRNA, ribozyme, double-stranded RNA and the like.

For example, the target sequence is not particularly limited, and includes a target sequence in nucleic acids to be detected by a known nucleic acid detection method. The target sequence is, for example, at least a part of sequence such as nucleic acids and other sequence features associated with specific genes in the organisms, nucleic acid mutations and other sequence features associated with genes that characterize individual organisms, nucleic acids associated with genes that characterize diseases and pathogens in the animals and plants, mutations on the nucleic acids, other sequence features and the like.

### (Nucleic acid detection method)

In the nucleic acid detection method disclosed in this specification, an oligonucleotide probe having a hybridization sequence with respect to a target sequence of nucleic acids present in a test sample and at least one covalent bonding group that is crosslinkable with a target base in the target sequence by light irradiation when it is specifically hybridized with the target sequence in the hybridization sequence is used. Hereinafter, the oligonucleotide probe will be described, and this detection method will be then described.

### (Oligonucleotide probe)

The oligonucleotide probe disclosed in this specification (hereinafter referred to as the present probe) is prepared as a single-stranded nucleic acid having a framework known as a natural nucleic acid or modified nucleic acid. Nucleic acid frameworks other than deoxyribose or ribose include, for example, known frameworks such as D-threoninol. The present probe has, for example, a framework in which deoxyribose and/or ribose, which may be modified, are linked by phosphodiester bonds or bonding with other similar linking groups. The present probe typically has, as bases, bases constituting natural DNA that can hybridize with DNA in the test sample, except for covalent bonding groups to be described below.

Like known oligonucleotide probes, the present probe has a hybridization sequence with respect to a target sequence. Here, the length of the target sequence is not particularly limited, and varies depending on applications and the type of a target nucleic acid, and may be. for example, about 10 mer or more and 300 mer or less, for example, 10 mer or more and 300 mer or less, for example, 10 mer or more and 200 mer or less, for example, 10 mer or more and 100 mer or less, for example. 10 mer or more and 50 mer or less, and for example, 5 mer or more and 50 mer or less. In addition, the length is, for example, 20 mer or more and 200 mer or less, for example, 20 mer or more and 100 mer or less, for example, 20 mer or more and 80 mer or less, for example. 20 mer or more and 70 mer or less. and for example. 20 mer or more and 50 mer or less. According to this detection method, even if the length of the hybridization sequence is 100 mer or 50 mer or less, an authentic crosslinked hybridization product is formed only in the case of complete matching, components other than the authentic crosslinked hybridization product are effectively removed in a denaturation step to be described below, and as a result, it is possible to secure a high S/N ratio.

The hybridization sequence has a degree of complementarity at which it can be specifically hybridized with a target sequence. As will be described below. since the present probe has at least one covalent bonding group that replaces a base in the hybridization sequence, the base sequence excluding at least one base corresponding to the position of the covalent bonding group has, for example, an identity of 90% or more, for example, an identity of 95% or more, for example, an identity of 98% or more, for example, an identity of 99% or more, for example, an identity of 100%, with respect to the target sequence. Here, the identity will be described below.

Here, those skilled in the art can set the target sequence and the hybridization sequence in appropriate consideration of Tm during hybridization in addition to the base length.

In the hybridization sequence, the present probe may have a target base in the target sequence when it is specifically hybridized with the target sequence in the hybridization sequence and at least one covalent bonding group that is crosslinkable by light irradiation. Such covalent bonding groups that are linked to a framework in the base sequence, for example, deoxyribose or ribose, are provided.

The covalent bonding groups are not particularly limited, and examples thereof include known crosslinkable functional groups having photocrosslinkability disclosed in Japanese Patent Application Publication No. 2016-145229, Japanese Patent Application Publication No. 2017-210448, Japanese Patent Application Publication No. 2019-26569 and the like. All of these form a crosslinked structure with a pyrimidine ring of the pyrimidine base at the base position adjacent to the 3' side of the base in the target sequence that pairs with the covalent bonding group (+1 position: the position shifted to the 3' side by one base from the paired base) by light irradiation.

For example, deoxyribonucleosides having covalent bonding groups disclosed in Japanese Patent Application Publication No. 2016-145299 are shown below. When the present probe is a DNA probe, it includes such nucleosides that are linked to adjacent nucleosides with the (1'-5')-phosphodiester bond.

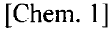

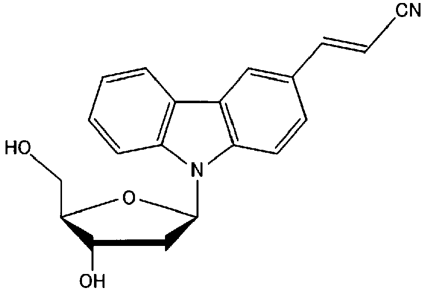

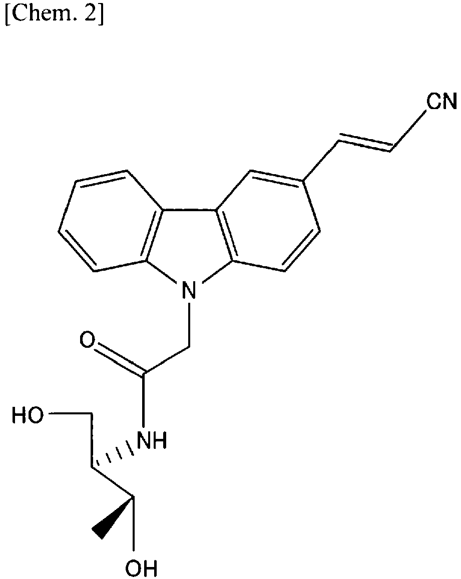

The pyrimidine bases forming a crosslinked structure are not particularly limited, and are thymine (T) or cytosine (C) for DNA and cytosine (C) or uracil (U) for RNA. Here, these bases may be modified as long as they can be photocrosslinked with covalent bonding groups, and examples thereof include 5-methylcytosine and 5-hydroxymethylcytosine.

For example, in the target sequence, the covalent bonding group is provided in a hybridization sequence so that it replaces any base that pairs with the base at the base position (-1 position) adjacent to the 5' side of the pyrimidine base in the target sequence. In other words, the covalent bonding group is provided so that it replaces any base at the base position (+1 position) adjacent to the 3' side of the purine base (adenine (A) or guanine (G)) in the hybridization sequence. In addition, the covalent bonding group is provided in the hybridization sequence so that it replaces any base that pairs with the base at the base position (+1 position) adjacent to the 3' side of the pyrimidine base in the target sequence. In other words, the covalent bonding group is provided so that it replaces any base at the base position (-1 position) adjacent to the 5' side of the purine base (adenine (A) or guanine(G)) in the hybridization sequence.

The covalent bonding group is provided in place of a base. for example, at a base-bonded part in the framework such as deoxyribose, ribose and D-threoninol.

Those skilled in the art can easily synthesize the present probe having a covalent bonding group in DNA or RNA, as described in Japanese Patent Application Publication No. 2016-145229, Japanese Patent Application Publication No. 2017-210448, Japanese Patent Application Publication No. 2019-26569, and the like, based on these publications and common technical knowledge and well-known techniques related to DNA synthesis techniques at the time of filing this application.

The number of covalent bonding groups in the present probe is not particularly limited, but varies depending on the length of the hybridization sequence or the type and sensitivity of the labeling element. For example, when the hybridization sequence has a length of about 10 to 100 mer. the number of covalent bonding groups is, for example, 1 or more and 10 or less. for example. 1 or more and 6 or less, for example, 1 or more and 5 or less, for example, 1 or more and 4 or less, for example. 1 or more and 3 or less, or for example, 1 or more and 2 or less. When one probe includes a part in which two or more covalent bonding groups are continuous, if an additional covalent bonding group is further provided, it is preferable to provide the additional covalent bonding group at a distance of at least 10 bases or more from the continuous covalent bonding group.

In addition, the position of the covalent bonding group in the present probe is not particularly limited, and is, for example, 5' end side and/or 3' end side, and may be a position at which inhibition of hybridization with respect to the target sequence of the present probe can be minimized. Typically, it is the 5' end side region or the 3' end side region. Here, the 5' end side region is, for example, within the number of bases of 20% of the total number of bases in the hybridization sequence from the terminal of the 5' end, and the 3' end side region is, for example, within the number of bases of 20% of the total number of bases in the hybridization sequence from the terminal of the 3' end. In addition, for example, the covalent bonding group may be inside the end on the 5 end side, and may be present inside by at least 1 or more on the 3' end side.

On the other hand, for example, if the hybridization sequence has a length of about 10 mer or more and 100 mer. the position of the covalent bonding group may be a center part of the hybridization sequence, that is, may be within a range exceeding 25% of the base length of the hybridization sequence from the 5' end and the 3' end of the present probe. As long as the hybridization sequence has a certain length or more, even if it is in the center part of the hybridization sequence, it does not inhibit specific hybridization with the target sequence, and it can form an authentic hybridization product and an authentic crosslinked hybridization product.

Regarding the present probe. for example, a plurality of (for example, about several to several tens of) probe sets having a hybridization sequence of about 10 to 100 mer with respect to a long target sequence, typically a target sequence exceeding several hundred of mer to several thousands of mer, may be prepared. Long probes have high sequence specificity, but generally have a poor S/N ratio, but the present probe of 10 to 100 mer has low sequence specificity, but photocrosslinked when completed matched, and then, according to denaturation, non-specific hybridization products or uncrosslinked intrinsic hybridization products can be removed. Therefore, even if a long target sequence is detected with a plurality of probes, it is possible to secure a high S/N ratio and false positive detection results can be avoided or sufficiently minimized. In addition, probes of about 10 to 100 mer are less costly for synthesis than long probes and favorable penetration into cells.

The present probe may include, as necessary, a labeling element. The labeling element may be a signal generating element that can generate a signal by itself or by excitation or the like, or a binding element that does not generate a signal by itself but can bind to a signal generating element that can bind to the signal generating element. Such a signal generating element and binding element are well known to those skilled in the art in this technical field, and as necessary, those skilled in the art can select a labeling element and apply it to the present probe.

### (Hybridization step)

This detection method may include a hybridization step in which the present probe is brought into contact with a target nucleic acid in a test sample to form an authentic hybridization product. In the hybridization step, when the target nucleic acid is brought into contact with the present probe, it is possible to obtain an authentic hybridization product in which the present probe and the target nucleic acid are specifically hybridized.

Conditions such as the temperature, pH. salt concentration, and time in the hybridization step can be appropriately set for the stringency according to the Tm of the present probe. the type of the test sample and the like. For example, when the temperature is set to the Tm of the present probe or higher, the stringency becomes high and the hybridization specificity is improved, but the hybridization efficiency tends to decrease. On the other hand, when the temperature is set to be lower than the Tm, the stringency becomes low and the hybridization specificity decreases, but the hybridization efficiency tends to improve. In addition, the stringency can be adjusted according to the salt concentration or the pH.

In this detection method, the authentic hybridization product is photocrosslinked in the subsequent light irradiation step, the denaturation step is additionally performed, and thus an authentic crosslinked hybridization product can be maintained with high selectivity. Therefore, the hybridization step does not necessarily have to be performed with high stringency. Therefore, the hybridization step can be performed, for example, at room temperature, which will be described below, or can be performed at 25°C or higher and 40°C or lower, for example. 30°C or higher and 40°C or lower.

Here, when the hybridization step is performed, it may be necessary to perform a pretreatment allowing hybridization between the target nucleic acid and the present probe to be performed according to the test sample or detection method used. The pretreatment is well known to those skilled in the art depending on the test sample, the detection method and the like, and those skilled in the art can determine the pretreatment method and hybridization conditions based on well-known techniques.

In the hybridization step. it is necessary to avoid photocrosslinking of covalent bonding groups of the present probe. Therefore, according to the type of covalent bonding groups, as necessary, the hybridization step is performed while light blocked.

Embodiments of the hybridization step vary depending on the nucleic acid detection method to which this detection method is applied. Such a nucleic acid detection method may be a detection method using a solid-phase object such as an array or strip on which the present probe is fixed or a detection method with ISH or FISH in which in situ hybridization is performed.

In situ hybridization can be performed on the collected cells. cultured cells, a biological tissue section and the like. Regarding a target to which in situ hybridization is applied, for example, the present probe can be supplied to cells collected from breast cancer patients, cultured cells thereof, or the inside of tissue sections collected from breast cancer patients. In addition, cells preserving Epstein-Barr virus (EBV) infection, cultured cells thereof, tissue sections collected from patients with malignant tumors caused by EBV-infected disease and the like may be exemplified.

The nucleic acid hybridization step further includes, for example, hybridization in a liquid phase, and hybridization by chromatography using a column or strip on which the present probe is fixed. All of these methods are well known at the time of filing this application. Those skilled in the art can perform an appropriate hybridization step according to the type of the detection method to be applied based on well-known techniques.

### (Light irradiation step)

This step is a step in which light is irradiated to the test sample after the hybridization step, and the covalent bonding group in the authentic hybridization product and the target sequence are crosslinked to form an authentic hybridization product having a crosslinked structure (in this specification, this is referred to as an authentic crosslinked hybridization product). In the previous hybridization step, authentic hybridization products as well as non-specific mishybridized products containing mismatches but hybridized can be formed.

In the light irradiation step, a hybridization sequence including covalent bonding groups is appropriately hybridized to the target sequence to form a crosslinked structure with high specificity with the pyrimidine base on the +1 position side of the paired base of the target sequence. If there is a mismatch, it is difficult to form a positional relationship in which the hybridization sequence and the pyrimidine base, which is a crosslinking target of the target sequence, can be crosslinked, and even if appropriate light irradiation is performed, a crosslinked structure is unlikely to be formed. Therefore, a crosslinked structure is specifically formed in the authentic hybridization product. Therefore, according to this detection method. false negative detection results can be minimized and accordingly, the examination sensitivity can increase.

Light irradiation conditions for forming a crosslinked structure are determined according to the type of covalent bonding groups used and the like. For example, when covalent bonding groups disclosed in Japanese Patent Application Publication No. 2016-145229 are used, light having a wavelength of 330 to 380 nm, 340 to 380 nm, and 350 to 380 nm can be used. In addition, when covalent bonding groups disclosed in Japanese Patent Application Publication No. 2019-26569 are used, for example, light having a wavelength of 350 to 600 nm, for example, 400 to 600 nm, for example, 400 to 550 nm, and for example. 400 to 450 nm, can be irradiated.

The temperature and time in the light irradiation step are not particularly limited, and may be, for example, 0°C to 50°C. for example, 0 to 40°C, for example, 0 to 30°C, for example, 0 to 20°C, for example, 0 to 10°C, or for example, 0 to 5°C. In addition, the time may be, for example, 1 to 120 seconds, or for example, 1 to 60 seconds.

Here, in the light irradiation step, since a light reaction is used, the pH, the salt concentration and the like are not particularly limited, and in addition to hybridization products and the like, the pH and salt concentration conditions in which biomaterials can be stably present can be used.

### (Denaturation step)

This step is a step in which denaturation conditions in which the authentic crosslinked hybridization product can be maintained and a non-specific hybridization product can be dissociated are applied to the test sample after the light irradiation step and the oligonucleotide probe derived from the non-specific hybridization product is separated. For such denaturation conditions, for example, various conditions in which the double strand such as DNA can be dissociated are applied.

According to this step, according to the above denaturation conditions, the non-specific hybridization product can be easily separated from the authentic crosslinked hybridization product and removed (washed). Conventionally, when there is no large difference in stability between the non-specific hybridization product and the authentic hybridization product, washing with high stringency may denaturate and wash the authentic hybridization product, and on the other hand, washing with low stringency may cause a problem of the non-specific hybridization product not being washed sufficiently. However, since the authentic crosslinked hybridization product has a crosslinked structure with covalent bonds, the product no longer dissociate under denaturation conditions in which the hybridization product with the base pair is dissociated. Therefore, even if denaturation conditions with high strength are applied, the authentic crosslinked hybridization product can be stored so that the denaturation conditions can be easily set, and only the authentic crosslinked hybridization product can be reliably maintained.

The denaturation step can be performed so that dissociation of hybridization of an uncrosslinked authentic hybridization product is additionally promoted, and the oligonucleotide probe derived from the uncrosslinked authentic hybridization product and the authentic crosslinked hybridization product are separated. According to this detection method, denaturation conditions with high strength in which crosslinked/uncrosslinked hybridization products rather than high/low stringency can be distinguished can also be used, and as a result, false positive detection results can be minimized, and accordingly, the examination specificity can be improved.

According to this detection method, as described above, false positive detection results are minimized, and as a result, accurately, the accuracy with which true positive and true negative can be detected can be improved. Even if this detection method is, for example, a method in which classification into a plurality of levels and evaluation are performed from the examination results based on the signal intensity, improvement in sensitivity, specificity, and accuracy can contribute to the accuracy and precision of the examination.

Conventionally, suitable stringency conditions for washing differ for each probe, and in this detection method, since it is only necessary to maintain the authentic crosslinked hybridization product. there is no need to set detailed conditions such as changing denaturation conditions for each probe or perform a plurality of operations under different denaturation conditions, and even if a plurality of present probes is applied at the same time, since the same denaturation conditions can be applied, it is also useful for a detection method of detecting a plurality of target nucleic acids at the same time.

In the denaturation step, the denaturation strength at which dissociation of the non-specific hybridization product and eventually the authentic hybridization product is promoted can be adjusted according to the temperature, the salt concentration, the pH and the like. in addition to the denaturant. When the denaturant strength and the denaturant concentration are higher, the temperature is higher, the pH is higher, and the salt concentration (ionic strength) is lower, the denaturation strength becomes stronger. For the denaturation conditions, various factors such as the denaturant, the temperature, the base and the pH can be used alone or two or more thereof can be used in combination.

For the denaturant, a known denaturant known to dissociate hybridization of DNA or RNA can be used. Although not particularly limited, for example, a solvent can be used as such a denaturant. Examples of solvents include aldehydes such as formamide and dimethylformamide, pyridines, piperidines, dimethylsulfoxide, tetrahydrofuran and acetonitrile.

When a solvent is used as a denaturation factor, the denaturation conditions can be adjusted according to not only the type of the denaturant but also the concentration of the denaturant and the diluent used at the same time. The solvent as a denaturant is not particularly limited, and a solution of, for example, 40 volume% or more, for example, 50 volume% or more, for example, 60 volume%, for example, 70 volume% or more, for example, 80 volume% or more, for example, 90 volume% or more, for example, 95 volume% or more, or for example, 100 volume% can be used. 70 volume% or more or 80 volume% or more is preferable. In addition, the diluent may be, for example, a biocompatible buffer solution such as a phosphate-buffered physiological saline solution, or an aqueous medium containing water with higher stringency (pure water, etc.). That is, an aqueous mixed solution mixture can be used. When a solvent is used as a denaturant, the strength of the denaturation conditions can be increased beyond expectations, depending on the type of the diluent, for example, if pure water is used in place of the buffer solution.

For example, when formamide (FA) is used as a solvent, it is possible to use an aqueous solution or buffer solution containing 50 volume% of FA. an aqueous solution or buffer solution containing 60 volume% of FA, an aqueous solution or buffer solution containing 70 volume% of FA, or an aqueous solution or buffer solution containing 80 volume% of FA. An aqueous solution or buffer solution containing 50 volume% or more of FA is preferable, an aqueous solution or buffer solution containing 60 volume% or more of FA is more preferable, and an aqueous solution or buffer solution containing 80 volume% or more of FA is still more preferable. In addition, when no FA is used, it is preferable to use a denaturant (denaturation solution) having a denaturation effect equal to or greater than that of an aqueous mixed solution mixture such as an aqueous solution or buffer solution containing, for example, at 60 volume% or more or more than 60 volume% of FA, or an aqueous mixed solution mixture such as an aqueous solution or buffer solution containing 80 volume% or more or more than 80 volume% of FA.

Examples of other denaturants include urea, alkylamines such as tetramethylammonium chloride. alkali such as NaOH, iodide ion, guanidine, peroxide, and thiosulfide. These denaturants can adjust the pH, the base and the like. These denaturants that are dissolved in a suitable solvent can be used. Examples of solvents include a biocompatible buffer solution such as a phosphate-buffered physiological saline solution, an aqueous medium such as pure water, and a solvent as a denaturant already described.

For the temperature conditions applied in the denaturation step, the denaturation step can be performed under the conditions alone, but all other denaturation factors involve temperature conditions. Therefore, the temperature conditions applied in the denaturation step are appropriately set according to the denaturation strength such as the type and degree of denaturation factors applied in the denaturation step. For example, for the denaturation factor, when, for example, a denaturant such as a solvent is used, the temperature condition to be applied may be the temperature of a place in which this detection method is performed without special temperature control, and the step can be performed at a temperature or higher (in this specification, the temperature is referred to as room temperature) at which special heating or cooling is not performed. In this specification, "room temperature" is, for example, 1°C or higher and 35°C or lower, for example, 1°C or higher and 30°C or lower, for example, 1°C or higher and 30°C or lower, for example, 10°C or higher and 30°C or lower, or for example, 15°C or higher and 25°C or lower. The denaturation step at room temperature is particularly suitable in that damage to a test sample such as tissues subjected to in situ hybridization is minimized, and is advantageous in that it does not require a special temperature control device or the like. The temperature condition in the denaturation step may be. for example, 20°C or higher, for example, 30°C or higher, for example, 35°C or higher, for example, 40°C or higher, or for example, 50°C or higher. On the other hand, the upper limit may be. for example, 70°C or lower or for example. 60°C or lower. The temperature condition may be. for example, a temperature exceeding room temperature, and may be 40°C or higher and 60°C or lower.

In addition, when the temperature is used as a denaturation factor, for example, 30°C or higher and 90°C or lower, for example. 40°C or higher and 90°C or lower, 50°C or higher and 90°C or lower, or 70°C or higher and 90°C or lower can be set. For example, when a high temperature condition of 70°C or higher and 90°C or lower is used, even if no denaturant is used, only the authentic crosslinked hybridization product can be maintained even with only water or a buffer solution.

Such a denaturation step can be performed by applying an appropriate combination of one, two or more denaturation factors to the hybridization product or the like. When the denaturation step is performed, dissociation of hybridization is promoted, probes constituting non-specific hybridization products are removed, and additionally, probes constituting authentic hybridization products may also be removed, and thus it is possible to reliably retain the authentic crosslinked hybridization product and easily remove the background and false positive factors.

In the authentic crosslinked hybridization product after the denaturation step, according to the embodiment of the hybridization step or the like, the detection step is appropriately performed. The detection step is typically performed by a known method using a labeling element that is pre-attached to the probe.

According to this detection method described above, non-specific hybridization products and authentic hybridization products can be removed without using a long probe for raising the Tm temperature and without depending on the stringency strength, and authentic crosslinked hybridization products can be detected with high accuracy.

### (Oligonucleotide probe)

The oligonucleotide probe for detecting nucleic acids disclosed in this specification can have the configuration as described above. Here, in the present probe, a hybridization sequence and at least one covalent bonding group are provided, an authentic crosslinked hybridization product is maintained, but a non-specific hybridization product can be selectively removed. Accordingly, in this detection method, it is possible to improve the detection accuracy and reproducibility. The present probe is also provided as a kit in combination with a denaturant useful in the denaturation step of this detection method. Such denaturants may include, for example, a denaturant having an effect equal to or greater than that of an aqueous mixed solution mixture containing 60 volume% or more of formamide.

### [Examples]

Hereinafter, in order to describe the present disclosure more specifically, examples as specific example will be described. The following examples are intended to illustrate the present disclosure, and do not limit the scope thereof.

### [Example 1]

### (Intracellular 28S rRNA FISH in tissue section collected from breast cancer patient)

Based on the human 28S rRNA base sequence, the following three types of hybridization sequences with different lengths were set so that they were crosslinked with thymine (T), which is a crosslinking target of the following covalent bonding group. The underlined bases are parts substituted with covalent bonding groups to be described below.
Hybridization sequence 1 (34 mer): tgctactaccaccaagatctgcacctgcggcggc (SEQ ID NO: 1)
Hybridization sequence 2 (25 mer): gctactaccaccaagatctgcacct (SEQ ID NO: 2)
Hybridization sequence 3 (17 mer): tgctactaccaccaaga (SEQ ID NO: 3)

In the above three types of sequences, one covalent bonding group (X) was introduced at the position shown below. In addition, three types of DNA oligonucleotide probes 1 to 3 whose 5' ends were modified with Cy3 were synthesized. Here, X is represented by the following structural formula

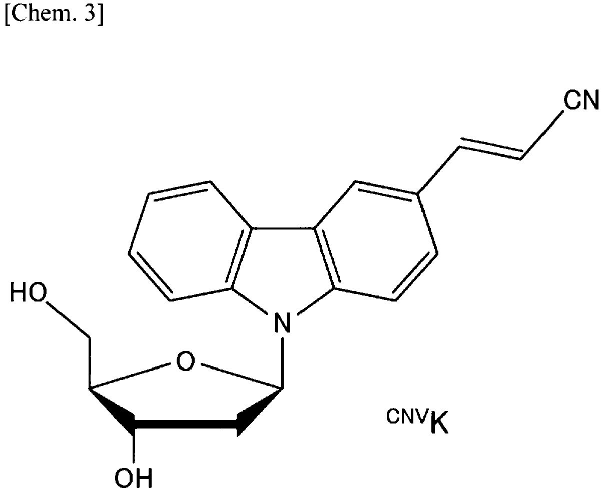

Hybridization sequence 1X (34 mer):
Cy3-tgctactaccaccaagatctgcaxctgcggcggc (SEQ ID NO: 4)
Hybridization sequence 2X (25 mer): Cy3-gctactaccaccaagatctgcaxct (SEQ ID NO: 5)
Hybridization sequence 3X (17 mer): Cy3-tgctactaccaccaxga (SEQ ID NO: 6)

Paraffin sections of tissue collected from breast cancer patients were deparaffinized and deproteinized according to general methods, and fixed with 4% paraformaldehyde and aldehyde was then neutralized. A probe solution prepared using a formamide-based buffer solution containing no blocking DNA so that the final concentration of the probe was 1 µg/ml was heated at 80°C for 3 minutes and then rapidly cooled on ice, the probe was single-stranded, 50 µl of the solution was added dropwise on the section, the sample was covered with a cover glass and left in an oven at 37°C for 1 hour, and hybridization was performed.

After the hybridization, the cover glass was removed. UV light having a wavelength of 366 nm was irradiated to the tissue sections 60 seconds, and immersion washing with 50 volume% formamide/SSC or 80 volume% formamide/SSC heated at 50°C three times, subsequently, with SSC twice, and with ultrapure water once for about 1 to 3 minutes were then performed, and sealing with glycerol was then performed.

When Cy3 signals were observed for these sections, regardless of the length of the probe, favorable signals could be confirmed in the cytoplasm for both 50 volume% FA denaturation and 80 volume% FA denaturation. Here, when a probe having no covalent bonding groups synthesized as a control was used, in 50 volume% FA denaturation, the probe was not removed but was maintained. However, in 80 volume% FA washing, the probe was completely washed, and almost no signal could be confirmed.

In addition, all of 34 mer, 25 mer and 17 mer probes 1 to 3 exhibited signals after 80 volume% FA denaturation, but the signal of the 17 mer probe 3 was the weakest, and the signals of the 34 mer and 25 mer probes 1 and 2 were almost equivalent. From the above, it was found that the hybridization sequence of about 20 mer or more was preferable. In addition, a DNA probe 4 having a hybridization sequence 1X' in which the third G from the 3' end was replaced with X at the X position in the hybridization sequence 1 (34 mer) was synthesized, and using DNA probes 1 and 4, tissue sections derived from the same breast cancer patients were simultaneously subjected to hybridization, light irradiation and 80 volume% FA washing in the same manner as described above, and the signals of the sections were observed. As a result, both signals were equivalent. From the above, it was found that the authentic crosslinked hybridization product could be sufficiently formed even if the covalent bonding group was not on the 3' end side.

In addition, between 50 volume% FA washing and 80 volume% FA washing, an image was clearer in 80 volume% FA washing. This was thought to be the result of improvement in the S/N ratio due to proper removal of the non-specific hybridization products or the like.

### [Example 2]

### (Examination of light irradiation conditions using 25 mer probe)

Using the probe 2 synthesized in Example 1, 28S rRNA FISH was performed with different UV irradiation times, and the effect of the irradiation time was evaluated. FISH was performed in the same manner as in Example 1 except that the light irradiation conditions were set to 0.1 seconds, 5 seconds, 60 seconds and 300 seconds.

As a result, clearly, there was a tendency for the signal intensity to increase with extension of the irradiation time. On the other hand, over 60 seconds or longer, the signal intensity did not increase significantly.

### [Example 3]

### (Examination of covalent bonding group)

Using the probe 2 synthesized in Example 1 and the probe 5 in which another covalent bonding group D having a D-threoninol framework represented by the following structural formula was introduced at the same position as the covalent bonding group X introduced in the probe 2. 28S rRNA FISH was performed according to Example 1, and the effect of the type of the covalent bonding group was evaluated. The light irradiation time was set to 5 seconds or 60 seconds, 80 volume% FA was used for washing, and FISH was performed in the same manner as in Example 1.

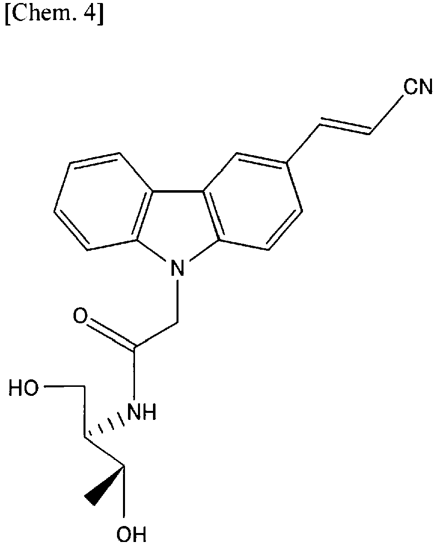

As a result, the signal of the probe 5 was stronger than that of the probe 2 at both the UV irradiation time of 5 seconds and 60 seconds. This was thought to be the result that the probe 5 into which a covalent bonding group D (CNV-D) whose main framework was D-threoninol was introduced formed authentic crosslinked hybridization products with 28S rRNA more efficiently than the probe 2 into which a covalent bonding group X (CNV-K) whose main framework was deoxyribose was introduced.

### [Example 4]

### (Satb2 protein mRNA CISH in cerebrum section collected from mice)

For Satb2mRNA (about 700 bases) as a target sequence, as shown in FIG. 1, 26 probes of about 20 mer (SEQ ID NO: 7 to 32) were designed, a covalent bonding group X was introduced in the same manner as in Example 1 so that it was photocrosslinkable with thymine (T) in the target sequence. In addition, DIG was added to the 5' end of the DNA probe.

A mouse brain frozen section was thawed at 37°C for 30 minutes and then immersed in 4% paraformaldehyde at 37°C for 10 minutes and post- fixed. After immersion in a TBS buffer solution was performed for 5 minutes twice, immersion in a 2 mg/ml glycine/PBS solution was performed for 30 minutes, aldehyde was then neutralized, and immersion in a 40 volume% deionized formamide/4×SCC solution at 55°C was performed for 30 minutes. Then, a hybridization solution containing 1 µg/ml of each of the above probes was heated at 80°C for 5 minutes, 50 µl of the solution was supplied onto the section, and hybridization was performed at 37°C for 1 hour.

Then, a slide on which the section was placed was placed on ice, and light having a wavelength of 366 nm (1,000 mV) was irradiated for 1 minute. In addition, immersion denaturation/washing in an 80 volume% formamide/TBS buffer solution at 50°C or an 80 volume% formamide aqueous solution at the same temperature was repeated for 3 minutes three times.

Then, immersion washing with a TBS buffer solution was performed for 3 minutes twice, immersion washing with the same buffer solution was performed for 1 minute once, 50 µl of a 200-fold diluted solution containing alkaline phosphatase-labeled antibodies was added dropwise, left at room temperature for 30 minutes, washing with a TBS buffer solution was performed for 3 minutes twice, washing with ultrapure water was performed for 1 minute twice. a BCIP/NBT solution was added, and after the sample was reacted in the dark for 60 minutes, washing with running water, dehydration, and sealing were performed.

FIG. 2 shows the results in which color development from blue to bluish purple was confirmed for these section samples. As shown in FIG. 2(B), when an 80 volume% formamide buffer solution was used, traces of Satb2-specific signal were observed in the cerebral cortex, but they were not clear. On the other hand, as shown in FIG. 2(A), when an 80 volume% formamide aqueous solution with a higher denaturation strength was used, clear Satb2-specific signals could be confirmed in the cerebral cortex with reduced non-specific signals.

### [Example 5]

### (HER2 protein mRNA CISH in breast cancer cultured cells)

It was designed such that the following two probes hybridized with the target sequence from Exon 15 to 17 in HER2mRNA. Here, in the probe, as X in the following sequence, a covalent bonding group X was introduced in the same manner as in Example 1 so that it was photocrosslinkable with uracil (U) or cytosine (C) in the target sequence, and DIG was added to the 5' end.
Probe 1: GTCCACACAGGAGTGGGTGCAXTT (SEQ ID NO: 33)
Probe 2: CGTCAGAGGGCTGGCTCTCTGXTC (SEQ ID NO: 34)

Two types of breast cancer cells (BT474: HER2 positive, BT20: HER2 negative) were cultured to create a cell block. After paraffin embedding, sliced slides were prepared. Immunostaining was performed on HER2 proteins, it was checked whether they were HER2 positive or negative, and ISH was then performed using the probes 1 and 2 having the covalent bonding group X.

Deparaftinization and hydrophilization were performed and the specimen was heated in an oven at 60°C to melt paraffin, and washing with Clear Plus (deparaffinizing agent, commercially available from Falma) 6 times, with 100% EtOH three times, with 95% EtOH twice, with 80% EtOH once, and additionally, with pure water 5 times were then performed. Additionally, a protein kinase solution was supplied, a treatment was performed at room temperature for 30 minutes, and washing with pure water was then performed twice. Then, the sample was immersed in 95% EtOH and dried with a hair dryer.

A hybridization solution containing 1 µg/ml of two types of probes (a TE buffer solution containing 40 volume% of formamide) was heated at 80°C for 3 minutes and then rapidly cooled, and 50 µl of the solution was supplied to the tissue and then the sample was covered with a cover glass and left in a thermostatic chamber at 55°C for 1 hour, and hybridization was performed.

After the hybridization. light having a wavelength of 366 nm (1,000 mV) was irradiated on ice for 1 minute, and the sample was then immersed in an 80 volume% formamide 2×SSC solution (55°C) for 3 minutes and denaturation/washing were performed.

Then, after washing with 2×SSC once and with TBS twice, an anti-DIG primary antibody solution was added dropwise, and left at room temperature for 30 minutes. After washing with TBS three times, an alkaline phosphatase-labeled polymer that specifically binds to primary antibodies was added dropwise and left at room temperature for 30 minutes. After washing with TBS three times, BCIPINBT was added dropwise, the reaction was performed in the dark overnight, washing with running water was then performed, dehydration was performed and sealing was then performed. FIG. 3 shows the results.

As shown in FIG. 3, in ISH using the probe having the covalent bonding group X, clear signals were confirmed in HER2 positive cells. On the other hand, in HER2 negative cells, no signals were confirmed. It was found that the results obtained with ISH matched the results of immunostaining performed in advance, and HER2mRNA could be specifically detected.

### [Example 6]

### (EBER (EB virus-produced small RNA)-ISH)[1]

It was designed such that the following two probes were hybridized with the target sequence of about 100 mer in EBER1. Here, the probe was synthesized using a unit used in Example 1 for uracil in the target sequence as X in the following sequence, and DIG was added to the 5' end. Here, probes of comparative examples were synthesized in the same manner except that X was replaced with guanine or adenine.
Probe 1: GTCTCCTCCCTAGCAAAXCC (SEQ ID NO: 35)
Probe 2: GTCTGGGAAGACAACCAXAG (SEQ ID NO: 36)

EB virus-infected tissues collected from gastric cancer patients were deparaffinized and hydrophilized, the specimen was heated in an oven at 60°C to melt paraffin, washing with Clear Plus (deparaffinizing agent, commercially available from Falma) 6 times, with 100% EtOH three times, with 95% EtOH twice, with 80% EtOH once, and additionally, with pure water 5 times were then performed. Additionally, a protein kinase solution was supplied, a treatment was performed at room temperature for 30 minutes, and washing with pure water was then performed twice. Then. the sample was immersed in 95% EtOH and dried with a hair dryer.

A hybridization solution containing 1 µg/ml of two types of probes (a TE buffer solution containing 40 volume% of formamide) was heated at 80°C for 3 minutes and then rapidly cooled, and 50 µl of the solution was supplied to the tissue and then the sample was covered with a cover glass and left at room temperature for 1 hour, and hybridization was performed. Hybridization was performed on the probe of the comparative example in the same manner.

After the hybridization. light having a wavelength of 366 nm (1,000 mV) was irradiate d on ice for 1 minute, and the sample was then immersed in a 50 volume% formamide 2×SSC solution (room temperature) and 80 volume% formamide 2×SSC solution (55°C) for 3 minutes and denaturation/washing were performed.

Then, washing with 2×SSC once and with TBS twice, and an alkaline phosphatase-labeled antibody solution was then added dropwise, and left at room temperature for 30 minutes. After washing with TBS twice and with ultrapure water twice, BCIP/NBT was added dropwise, the reaction was performed in the dark for 60 minutes, washing with running water was then performed, dehydration was performed, and sealing with Glycergel was then performed. FIG. 4 shows the results.

As shown in FIG. 4, when the probe having the covalent bonding group X was used. color development occurred with both the 50 volume% formamide 2×SSC (room temperature) and the 80 volume% formamide 2×SSC solution (55°C), but the area stained with 80 volume% formamide was smaller. On the other hand, in the case of the DNA probe of the comparative example, color development occurred with 50 volume% formamide 2×SSC (room temperature), but no color development occurred with the 80 volume% formamide 2×SSC solution (55°C). From the above, it was found that, according to denaturation with higher strength denaturation conditions of 80 volume% formamide 2×SSC solution (55°C), only the crosslinked intrinsic hybridization product was detected.

From the above, the denaturation resistances of the probe having the covalent bonding group X and the probe containing no X were checked. It was found that, in order to detect the target sequence with high accuracy. the probes having covalent bonding groups and high-strength denaturation conditions were necessary.

### [Example 7]

### (EBER (EB virus-produced small RNA)-ISH)[2]

As in Example 6, for the probe having the covalent bonding group X and a commercially available EBER detection kit (Dako, PNA probe), the detection performance was evaluated. Regarding the probe having the covalent bonding group X, in denaturation conditions, 80 volume% formamide 2×SSC solution (room temperature) was used, and other operations were the same as in Example 6.

For the PNA probe kit, the appended protocol was applied. Here, according to the protocol, hybridization was performed at 55°C for 30 minutes, washing was performed using a stringent buffer solution at 55°C. and a color development reaction with alkaline phosphatase was used.

Sections treated with the probe having the covalent bonding group X and the PNA probe kit were evaluated. FIG. 5 shows the results. As shown in FIG. 5, in the probe having the covalent bonding group X, even though it was treated with hybridization at room temperature and denaturation at room temperature, clear staining was confirmed. In addition, in the PNA probe kit, according to hybridization at 55°C and washing at 55°C, the detection results equivalent to those of the probe having the covalent bonding group X were obtained. However, in the PNA kit, in hybridization at room temperature, the background was too high, and specific detection was not possible for detection.

From the above, it was found that, using the probe having the covalent bonding group X, it was possible to easily control the temperature and detect a target nucleic acid with high accuracy.

### Sequence Listing Free Text

SEQ ID NO: 1 to 36: probe

### [Sequence listing]

### Citation List

Patent Literature 1: Japanese Patent Application Publication No. 2015-142574

## Claims

1. A nucleic acid detection method, comprising:
hybridizing by bringing an oligonucleotide probe into contact with a nucleic acid present in a test sample, wherein the oligonucleotide probe has a hybridization sequence with respect to a target sequence of the nucleic acid and has at least one covalent bonding group that is crosslinkable with a target base in the target sequence by light irradiation when being specifically hybridized with the target sequence in the hybridization sequence, thereby forming an authentic hybridization product;
irradiating light to the test sample after the hybridizing, wherein the covalent bonding group in the authentic hybridization product and the target sequence are crosslinked, thereby forming the authentic hybridization product having a crosslinked structure: and
denaturating the test sample after the irradiating by applying a denaturating condition in which the authentic crosslinked hybridization product is able to be maintained and a non-specific hybridization product is able to be dissociated and separating the oligonucleotide probe derived from the non-specific hybridization product.

2. The method according to claim 1,
wherein the denaturating comprises further promotion of dissociation of hybridization of the authentic hybridization product and separation of the oligonucleotide probe, derived from the authentic hybridization product, and the authentic crosslinked hybridization product.

3. The method according to claim 1 or 2,
wherein the hybridizing comprises performing in situ hybridization.

4. The method according to claim 3,
wherein the hybridizing comprises supplying the oligonucleotide probe into tissue sections collected from a cultured cell, a cancer patient or the like.

5. The method according to any one of claims 1 to 4,
wherein the denaturating comprises denaturation of the test sample with an aqueous solution mixture containing formamide at a concentration exceeding 60 volume% after the hybridizing.

6. The method according to any one of claims 1 to 5,
wherein the denaturating comprises denaturation using a liquid at room temperature or higher.

7. The method according to any one of claims 1 to 6.
wherein the denaturating comprises denaturation with a denaturation solution containing a denaturant other than formamide.

8. The method according to claim 7,
wherein the denaturant other than formamide is tetramethylammonium chloride.

9. The method according to any one of claims 1 to 8,
wherein the irradiating comprises irradiation of the light having a maximum wavelength of 340 to 380 nm.

10. The method according to any one of claims 1 to 9,
wherein the hybridization sequence has 5 bases or more and 200 bases or less.

11. The method according to any one of claims 1 to 10,
wherein the target sequence comprises an RNA sequence in human HER2 protein mRNA.

12. The method according to any one of claims 1 to 10.
wherein the target sequence comprises an RNA sequence in Satb2 protein mRNA.

13. The method according to any one of claims 1 to 10.
wherein the target sequence comprises an RNA sequence in EBER small RNA.

14. The method according to any one of claims 1 to 10,
wherein the target sequence comprises an RNA sequence in 28S rRNA.

15. The method according to any one of claims 1 to 14,
wherein the denaturating comprises denaturation of the test sample with an aqueous solution mixture containing 80 volume% of formamide after the hybridizing.

16. An oligonucleotide probe for detecting nucleic acid, comprising
a hybridization sequence that is able to hybridize with a target sequence of the nucleic acid, and at least one covalent bonding group that is crosslinkable with a target base in the target sequence by light irradiation when being specifically hybridized with the target sequence in the hybridization sequence.
wherein the hybridization sequence and at least one covalent bonding group are configured such that an authentic cross-linked hybridization product in which the hybridization sequence and the target sequence are hybridized and crosslinked by the light irradiation is able to be maintained but a non-specific hybridization product hybridization product is able to be selectively removed.

17. A labeled oligonucleotide detection kit including the oligonucleotide probe according to claim 16 and a denaturant having an effect equal to or greater than that of an aqueous mixed solution mixture containing formamide at a concentration exceeding 60 volume%.
